# EUROPÄISCHE PATENTSCHRIFT

(11) **EP 0 857 722 B1**
(45) Veröffentlichungstag und Bekanntmachung des Hinweises auf die Patenterteilung: **17.07.2002**
(21) Anmeldenummer: 98100946.7
(22) Anmeldetag: 21.01.1998
(51) Int. Cl.: C07D 311/72

(54) **Verfahren zur Herstellung von Tocopherylcarbonsäureestern oder Tocotrienylestern durch säurekatalysierte Umsetzung mit Carbonsäuren**
Process for the preparation of tocopherylcarboxylic acid esters or tocotrienyl esters by acid catalysed reaction with carboxylic acids
Procédé de préparation d'esters d'acide tocopherylcarboxyliques ou de tocotrienylesters par réaction catalysée acide avec des acides carboxiliques

(30) Priorität: 07.02.1997 DE 19704619
(43) Veröffentlichungstag der Anmeldung: 12.08.1998
(73) Patentinhaber: BASF AKTIENGESELLSCHAFT, 67056 Ludwigshafen (DE)
(72) Erfinder: von dem Bussche-Hünnefeld, Joanna Linda, Dr., 68623 Lampertheim (DE); Jaedicke, Hagen, Dr., 67069 Ludwigshafen (DE); Harms, Guido, Dr., 67117 Limburgerhof (DE); Laas, Harald, Dr., 67133 Maxdorf (DE)

(56) Entgegenhaltungen:
- WO-A-97/28151
- CHEMICAL ABSTRACTS, vol. 99, no. 30, 1983 Columbus, Ohio, US; abstract no. 5879x, Seite 539; Spalte 1; XP002065418 & JP 57 193 473 A (MIDORI KAGAKU)

## Beschreibung

Die Erfindung betrifft ein Verfahren zur Herstellung von Tocopherylestern oder Tocotrienylestern durch säurekatalysierte Veresterung von einem Tocopherol oder einem Tocotrienol in einem Lösungsmittel bei erhöhter Temperatur mit der entsprechenden Carbonsäure, insbesondere mit Sorbinsäure.

Unter einem Tocopherol verstehen wir erfindungsgemäß eine Verbindung der allgemeinen Formel I 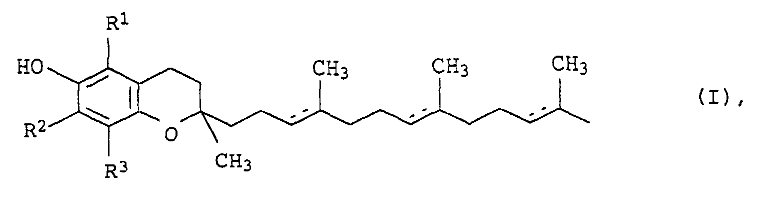
in der R¹ bis R³ für H oder Methyl stehen und die Seitenkette gesättigt ist,
unter einem Tocotrienol entsprechende Verbindungen, in denen die gestrichelten Linien eine weitere Bindung bedeuten.

Von besonderem Interesse ist das Verfahren für die Herstellung von Carbonsäureestern des α-Tocopherols, d.h. einer Verbindung, in der R¹ bis R³ eine Methylgruppe bedeuten und die Seitenkette gesättigt ist. α-Tocopherol besitzt die höchste Vitamin-E-Aktivität.

α-Tocopherol (Vitamin E; VE) hat in letzter Zeit große Bedeutung in der Tierernährung und als Nahrungsergänzungsmittel erlangt als wichtigstes Lipid-lösliches biologisches Antioxidant, und wegen seiner zusätzlichen biologischen Wirkung. Da die freien Tocopherole an der Luft sehr leicht oxidiert werden, verwendet man sie bevorzugt in Form ihrer Ester mit Carbonsäuren, welche bei Raumtemperatur relativ stabil gegenüber atmosphärischem Sauerstoff sind. Wie aus der Formel I zu ersehen ist, handelt es sich bei Tocopherolen um Verbindungen mit einer sterisch gehinderten phenolischen Hydroxylgruppe und somit um eine äußerst schwierig veresterbare Hydroxylgruppe.

Schon von Phenolen weiß man, daß sie zwar gut mit Säurechloriden oder Säureanhydriden, nicht aber mit Carbonsäuren zu Phenolestern reagieren (vgl. Brockhaus Chemie, VEB F.A., Brockhaus Verlag Leipzig, 5. Auflage, 1987, Band 2/L-Z, Seite 846-847, linke Spalte, Absatz 3 oder DBP 10 35 960). Gleiches gilt in verstärktem Maße für die Tocopherole und Tocotrienole mit ihrer sterisch gehinderten OH-Gruppe. So wird der wichtigste Tocopherylester, das Tocopherylacetat, technisch ausschließlich durch Umsetzung von Tocopherol mit Acetanhydrid hergestellt.

Eine Übersicht über Verfahren zur Herstellung von Estern von α-Tocopherol mit aliphatischen oder aromatischen Carbonsäuren findet sich in Jzvestiya vysshikh uchebnykh zavedenii, Khimiya i khimicheskaya tekhnologiya, 1991, 34(11), Seiten 3-26, insbesondere 4-9. Gemäß Seite (S.) 4-5 von loc. cit. werden Alkylcarbonate durch Umsetzen von VE mit Phosgen und anschließend mit Alkanolen in Gegenwart von Pyridin oder durch Umsetzen von VE mit Säurechloriden der Alkylcarbonsäuren hergestellt. Gemäß S. 5 von loc. cit. werden auch das Palmitat, Stearat, Propionat, Caprat und das p-Nitrobenzoat durch Umsetzen von VE mit den entsprechenden Säurechloriden in Gegenwart von Pyridin bei 50 bis 62°C hergestellt. Palmitat und Stearat können gemäß S. 5 von loc. cit. zwar auch durch Umsetzen von VE mit Palmitinsäure oder Stearinsäure hergestellt werden, dies aber nur in Gegenwart teurer exotischer Kondensationsmittel wie Carbonyldiimidazolen oder Thionyldiimidazolen. Gemäß S. 5 unten von loc. cit. werden Succinate, Malonate, Glutarate und Phthalate durch Umsetzen von VE mit einem Grignardreagenz und anschließend mit den Anhydriden der entsprechenden Säuren hergestellt. Gemäß S. 6 von loc. cit. werden das Malonat und das Phthalat durch Umsetzen von VE mit Malonsäureanhydrid bzw. Phthalsäure in Gegenwart von großen Mengen ZnCl₂ als wasserbindendem Mittel hergestellt. Gemäß S. 8 von loc cit. wird α-Tocopherylmethoxypolyoxyethylenacetat durch Umsetzten von VE mit Methoxypolyoxyethylenessigsäureanhydrid in Gegenwart von p-Toluolsulfonsäure (6 h) bzw. in Pyridin (6 Tage) hergestellt. Gemäß S. 8 von loc. cit. wird α-Tocopheryl-p-chlorphenoxyisobuttersäureester, welcher antiatherosklerotische Aktivität aufweist, durch Umsetzen von VE mit p-Chlorphenoxybuttersäure in Gegenwart von teuren Carbonyldiimidazolen oder mit dem entsprechenden Säurechlorid in Gegenwart von Pyridin oder aber durch Umsetzen von VE mit Natriummethylat in Methanol und Umsetzen des erhaltenen Natriumsalzes von VE mit p-Chlorphenoxyisobuttersäurechlorid hergestellt. Gemäß S. 8 von loc. cit. wird α-Tocopherylpivalat durch Kondensation von VE mit Pivalinsäure in Gegenwart von großen Mengen Pyrophosphat oder unter dem Einfluß von Enzymen hergestellt. Gemäß S. 8 unten von loc. cit. wird Tocopherylsalicylat durch Kondensation von VE mit Salicylsäure in Gegenwart von großen Mengen Tetraalkylpyrophosphat hergestellt. Gemäß S. 9 oben von loc. cit. werden α-Tocopherylcinnamat und α-Tocopherylferulat durch Umsetzen der entsprechenden Säurechloride hergestellt.

Gemäß S. 9 von loc. cit.,werden die als Verbindungen mit antiatherosklerotischer Wirkung begehrten α-Tocopherylester von Linolsäure, Ölsäure und Arachidonsäure durch Umsetzen von VE mit den genannten Säuren und Säurechloriden oder Anhydriden der genannten Säuren in Gegenwart von großen Mengen Tetraalkylpyrophosphat oder anderen Säurefängern oder aber neuerdings durch Umsetzen von VE mit den entsprechenden Säuren in Gegenwart von teuren Carbonyldiimidazolen oder Thionyldiimidazolen hergestellt.

Ein weiteres medizinisch interessantes α-Tocopherylderivat ist α-Tocopherylsorbat, welches als Sonnenschutzmittel von Interesse ist. Es wird gemäß EP 313 303 B1 durch Umsetzen von 1 Mol VE mit 4,3 Mol eines Polyphosphatesters und anschließendes 16-stündiges Rühren mit 1 Mol Sorbinsäure hergestellt.

Als weitere Anwendung für Tocopherylsorbat wird sein Einsatz als Bestandteil dermatologischer Kompositionen zur Anti-Akne-Behandlung beschrieben (vgl. US 5, 545,407)

Wie dem genannten Stand der Technik zu entnehmen ist, wird die Herstellung von α-Tocopherylestern nach dem Stand der Technik meist durch Umsetzen von VE mit Carbonsäurechloriden oder Carbonsäureanhydriden durchgeführt. Nachteilig hieran ist, daß die Säurechloride oder Anhydride vorher auf aufwendige Weise hergestellt werden müssen, daß das Arbeiten mit Säurechloriden technisch recht aufwendig ist und daß in vielen Fällen in Pyridin gearbeitet werden muß, was wegen seiner unangenehmen Eigenschaften (u. a. des Geruchs) bei Vitaminderivaten nicht vorteilhaft ist.

In den Fällen, wo VE nach dem Stand der Technik mit den Carbonsäuren selbst verestert wird, ist die Mitverwendung mehrfach molarer Mengen an wasserbindenden Mitteln wie Polyphosphatestern, Tetraalkylpyrophosphat oder Zinkchlorid oder aber die Gegenwart von teuren Kondensationsmitteln wie Carbonyldiimidazolen oder Thionyldiimidazolen notwendig. Die Mitverwendung von Polyphosphatestern und Tetraalkylpyrophosphat ist wegen der aufwendigen Herstellung derselben in technischem Maßstab nicht geeignet. Wie EP 313 303 zu entnehmen ist, ist z. B. die Herstellung von Polyphosphatestern durch die Verwendung von dem sehr hygroskopischen P₂O₅, dem gefährlichen Diethylether sowie von Chloroform und die lange Reaktionszeit von 48 Stunden technisch problematisch.

Bei Verwendung von Zinkchlorid und den aus ihrer Herstellung Chloridionen enthaltenden Imidazolen werden höchste Ansprüche an das Reaktormaterial und dadurch hohe Kosten verursacht.

Es war daher die Aufgabe der Erfindung, ein Verfahren zur Veresterung von Tocopherolen und Tocotrienolen zu entwickeln, bei dem die Tocopherole oder Tocotrienole mit den entsprechenden Carbonsäuren auf einfache und auch technisch sinnvolle Weise verestert werden können, ohne daß ein großer Überschuß an einem wasserbindenden Mittel oder aber teure exotische Kondensationsmittel wie Carbonyldiimidazol oder Thionyldiimidazole mitverwendet werden müssen.

Gegenstand der Erfindung ist dementsprechend ein Verfahren zur Herstellung von Tocopherylestern oder Tocotrienylestern durch säurekatalysierte Veresterung von einem Tocopherol oder einem Tocotrienol in einem Lösungsmittel unter Rühren bei erhöhter Temperatur, das dadurch gekennzeichnet ist, daß man
a) die Veresterung mit der entsprechenden Carbonsäure durchführt,
b) die Veresterung mit 2,5 bis 6 Mol, vorzugsweise 3 bis 5 Mol, der Carbonsäure in einem aliphatischen, cycloaliphatischen oder aromatischen Kohlenwasserstoff mit einem Siedebereich von 80 bis 200°C, vorzugsweise 110 bis 130°C,
   oder aber mit 1,0 bis 2,5 Mol, vorzugsweise 1 bis 1,5 Mol, der Carbonsäure in einem Gemisch aus einem aliphatischen oder cycloaliphatischen Kohlenwasserstoff mit einem Siedebereich von 80 bis 200°C und einem cyclischen Carbonat der allgemeinen Formel II oder einem γ-Lacton der allgemeinen Formel III 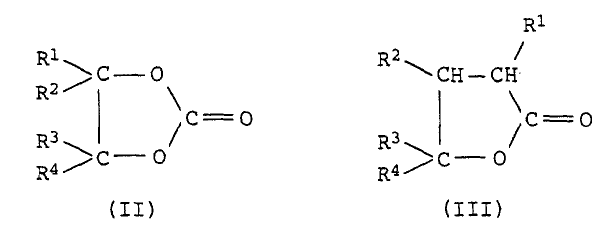 in denen die Reste R¹, R² und R³ für H oder C₁- bis C₄-Alkyl stehen und R⁴ für H, C₁- bis C₄-Alkyl, Phenyl oder Methoxymethyl steht, als Lösungsmittel durchführt,
c) eine nicht oxidierende starke anorganische oder organische Säure, vorzugsweise Schwefelsäure, Boroxalsäure oder Benzoloder Toluolsulfonsäuren in katalytischen Mengen als sauren Katalysator verwendet und
d) das bei der Reaktion gebildete Wasser während der Umsetzung fortlaufend azeotrop abdestilliert.

Überraschenderweise bleiben das cyclische Carbonat bzw. das γ-Lacton unter den Reaktionsbedingungen stabil und gehen nicht verloren.

Als Carbonsäuren, die mit einem Tocopherol oder Tocotrienol nach dem erfindungsgemäßen Verfahren verestert werden können, seien beispielsweise genannt:
Essigsäure, Propionsäure, 1-n-Hexansäure, Capronsäure, Sorbinsäure, Palmitinsäure, Stearinsäure, Ölsäure, Linolsäure und Arachidonsäure.

Besonders von Interesse ist das erfindungsgemäße Verfahren für die Umsetzung von einem Tocopherol oder einem Tocotrienol mit Essigsäure, Propionsäure, Sorbinsäure, Palmitinsäure, Stearinsäure, Ölsäure oder Linolsäure als Carbonsäure.

Als sauren Katalysator für die erfindungsgemäße Veresterungsreaktion kann man eine nichtoxidierende starke anorganische oder organische Säure, d. h. eine Säure mit einem pkₐ-Wert von etwa -6 bis 3 verwenden. Als besonders geeignete Säuren seien beispielsweise Schwefelsäure, Boroxalsäure (d. h. ein etwa äquimolares Gemisch aus Borsäure und Oxalsäure), eine Toluolsulfonsäure oder eine Benzolsulfonsäure genannt. Die Säuren verwendet man im allgemeinen in Mengen von 0,001 Mol bis 0,2 Mol Katalysatorsäure pro Mol Tocopherol oder Tocotrienol. Besonders vorteilhaft gestaltet sich das erfindungsgemäße Verfahren, wenn man zur Veresterung 0,004 bis 0,1 Mol, Schwefelsäure pro Mol Tocopherol oder Tocotrienol als sauren Katalysator verwendet.

Als erfindungsgemäß einsetzbare aliphatische Kohlenwasserstoffe mit einem Siedebereich von 80 bis 200°C, vorzugsweise 110 bis 130°C seien genannt, Heptane und Octane, insbesondere n-Heptan oder Gemische aus Kohlenwasserstoffen, wie Hexanen, Heptanen, Octanen und/oder Nonanen.

Als für das erfindungsgemäße Verfahren geeigneter cycloaliphatischer Kohlenwasserstoff sei Cyclohexan genannt.

Als geeignete aromatische Kohlenwasserstoffe für eine Verwendung in einem einphasigen System seien Benzol und Toluol genannt.

Verwendet man ausschließlich Kohlenwasserstoffe, d. h. ein einphasiges System, als Lösungsmittel, so muß in den meisten Fällen die Carbonsäure in hohem molarem Überschuß verwendet werden. Man verwendet dann die Carbonsäure mit Vorteil in Mengen von etwa 3 bis 5 Mol Carbonsäure pro Mol Tocopherol oder Tocotrienol. Mit Hilfe des als Lösungsmittel verwendeten Kohlenwasserstoffs kann das bei der Veresterungsreaktion gebildete Wasser fortlaufend azeotrop abdestilliert und in einem Wasserabscheider abgetrennt werden. Die überschüssige Carbonsäure kann z. B. im Falle der Sorbinsäure nach Beendigung der Umsetzung und Abkühlen des Reaktionsgemisches durch Auskristallisieren abgetrennt werden.

Verwendet man für die erfindungsgemäße Veresterung ein zweiphasiges Gemisch aus einem aliphatischen Kohlenwasserstoff und einem 5-Ring-Carbonat der allgemeinen Formel II oder einem γ-Lacton der allgemeinen Formel III, dann braucht man die Carbonsäure im allgemeinen nur in äquimolaren Mengen oder aber in einem geringen Überschuß (etwa bis 100 Mol.-% Überschuß) einzusetzen.

Bei Verwendung von Gemischen aus einem geeigneten Kohlenwasserstoff und einem 5-Ring-Carbonat oder 5-Ring-Lacton wird das bei der Veresterung gebildete Wasser in einem ternären azeotropen Gemisch abdestilliert. In dem aufgesetzten Wasserabscheider wird dann die untere, das Wasser in Form einer Lösung in dem Carbonat oder dem γ-Lacton enthaltende Phase abgetrennt und der Kohlenwasserstoff fortlaufend in das Reaktionsgefäß zurückfließen lassen. Das abdestillierte 5-Ring-Carbonat oder γ-Lacton muß im Reaktionsgefäß ständig durch neues, d. h. wasserfreies Carbonat oder γ-Lacton ersetzt werden. Ein großer Vorteil dieser Verfahrensvariante ist, daß nicht große Überschüsse an Carbonsäuren abgetrennt werden müssen und der erhaltene Tocopherylester oder Tocotrienylester aus der als Oberphase leicht abtrennbaren Kohlenwasserstofflösung in sehr sauberer Form isoliert werden kann. Das 5-Ring-Carbonat oder γ-Lacton der Unterphase kann nach Aufarbeitung für weitere Veresterungen verwendet werden.

Von ganz besonderer Bedeutung ist das erfindungsgemäße Verfahren für die Herstellung von dem als Lichtschutzmittel oder zur Anti-Akne-Behandlung begehrten α-Tocopherylsorbat, da die Veresterung von Tocopherolen mit ungesättigten Carbonsäuren ein besonderes Problem darstellt. Auch das für die kosmetische Anwendung besonders interessante all-E-Tocopherylsorbat kann mit Hilfe des erfindungsgemäßen Verfahrens sehr vorteilhaft hergestellt werden, ohne daß die Doppelbindungen im Tocotrienolteil oder im Sorbinsäureteil isomerisiert werden. Daher soll das erfindungsgemäße Verfahren im folgenden anhand der Veresterung von VE mit Sorbinsäure verdeutlicht werden.

Tocopherylsorbat kann nach dem erfindungsgemäßen Verfahren vorteilhaft hergestellt werden, wenn man zur Veresterung von VE
a) 2,5 bis 6, vorzugsweise 4 bis 5 Mol, Sorbinsäure pro Mol Tocopherol verwendet und
b) die Umsetzung in einem Kohlenwasserstoff mit einem Siedebereich von 80 bis 200°C bei Temperaturen von 110 bis 130°C durchführt.

Zur Herstellung von im wesentlichen all-E-Tocopherylsorbat ist es bei dem erfindungsgemäßen Verfahren vorteilhaft, wenn man
a) 4,5 bis 5,5 Mol Sorbinsäure pro Mol all-E-Tocopherol verwendet und
b) die Umsetzung in Petroleumbenzin mit einem Siedebereich von etwa 80 bis 140°C bei Temperaturen von 113°C bis 120°C durchführt.

Zur Isolierung des Tocopherylsorbats ist es bei dieser Verfahrensvariante vorteilhaft, wenn man das Reaktionsgemisch nach der Veresterungsreaktion auf Temperaturen von 0 bis 5°C abkühlt, und die dabei auskristallisierende überschüssige Sorbinsäure abfiltriert, bevor das Reaktionsgemisch in an sich bekannter Weise aufgearbeitet wird.

Eine weitere vorteilhafte Variante des erfindungsgemäßen Verfahrens, besteht darin, daß man zur Herstellung von Tocopherylsorbat
a) etwa 1,0 bis 2,5 Mol Sorbinsäure pro Mol Tocopherol verwendet und
b) die Umsetzung in einem Gemisch aus einem aliphatischen oder cycloaliphatischen Kohlenwasserstoff mit einem Siedebereich von 80 bis 200°C und Propylencarbonat oder γ-Butyrolacton durchführt.

Ganz besonders vorteilhaft gestaltet sich das erfindungsgemäße Verfahren, wenn man zur Herstellung von Tocopherylsorbat
a) etwa 1,5 Mol Sorbinsäure pro Mol Tocopherol verwendet und
b) die Umsetzung in einem Gemisch aus Octan und 1,2-Propylen-carbonat bei Temperaturen von etwa 110 bis 130°C durchführt.

Zur Durchführung dieser Verfahrensvariante geht man mit Vorteil so vor, daß man das Tocopherol und die Sorbinsäure in dem Gemisch aus Octan und 1,2-Propylen-carbonat oder γ-Butyrolacton vorlegt, konzentrierte Schwefelsäure als Katalysator zutropft, dann das Reaktionsgemisch für 3 bis etwa 15 Stunden zum Sieden unter Rückfluß erhitzt, wobei das bei der Reaktion gebildete Wasser azeotrop abdestilliert und in einem Abscheider fortlaufend in Form einer als Unterphase anfallenden Lösung in Propylencarbonat oder γ-Butyrolacton aus dem aufgefangenen Kondensat abgetrennt und das abdestillierte Propylencarbonat oder γ-Butyrolacton im Reaktionsgefäß durch wasserfreies ersetzt wird, und letztlich das gewünschte Tocopherylsorbat aus der Octanphase in an sich bekannter Weise isoliert.

Die folgenden Beispiele sollen das erfindungsgemäße Verfahren veranschaulichen.

### Beispiel 1

In einem 4-1-Scale-up-Gefäß mit Anker-Rührer wurden 228 g (0,496 mol) d,l-α-Tocopherol (Reinheit >96 %) und 278 g (2,48 Mol) ***all-E*** Sorbinsäure in 1,5 l Petrolbenzin vom Kp-Bereich 100 bis 140°C gelöst und hierzu unter kräftigem Rühren (200 U/min) innerhalb von 10 Minuten (min) 5 g (ca. 0,05 mol) konzentrierte (konz.) Schwefelsäure zugetropft. Das Reaktionsgemisch wurde anschließend bei 145°C Heizöltemperatur zum Sieden unter Rückfluß erhitzt (Innentemperatur ca. 110 bis 120°C) und der Ansatz 9 Stunden (h) unter Rühren und Auskreisen von Wasser (W) bei dieser Temperatur gehalten. Nach dieser Zeit lag der durch Gaschromatographie (GC) bestimmte Gehalt an unumgesetztem Tocopherol bei 1 bis 1,5 %.

Anschließend wurde das Reaktionsgemisch auf Temperaturen von 0 bis 5°C abgekühlt, die dabei auskristallisierende überschüssige Sorbinsäure abfiltriert und das resultierende Filtrat weiter aufgearbeitet. Dazu wurde es zunächst mit 100 ml W versetzt, mit 18 ml 25%iger Natronlauge auf einen pH-Wert von 7-8 gestellt und anschließend wurden die Phasen getrennt, wobei durch vorsichtiges Waschen eine Emulsionsbildung von vornherein vermieden wurde. Nach der basischen Wäsche wurde noch zweimal mit je 500 ml W gewaschen und anschließend die organische Phase eingeengt, wobei ein dunkelbraunes Öl erhalten wurde. Dieses wurde zur weiteren Aufreinigung in einer Molekulardestillation bei 0,03 bis 0,05 bar und 200 bis 209°C destilliert. Durch diese Destillation wurde der Gehalt an freiem Tocopherol bis auf < 1 % zurückgedrängt.

Die Ausbeute an hellgelbem all-E-α-Tocopherolsorbat betrug 81 % der Theorie, bezogen auf eingesetztes Tocopherol.

### Beispiel 2

### Technikumsbeispiel mit 3 Äquivalenten Sorbinsäure

In einem 250 Liter Technikumsrührkessel aus Stahl-Email mit Ankerrührer wurden 33,3 kg (77,3 mol) d,l-α-Tocopherol (Reinheit > 96 %) und 25,2 kg (224,7 mol) all-E-Sorbinsäure in 150 l Petroleumbenzin vom Kp 100 - 140°C gelöst und unter Rühren (1/80 min) zum Sieden unter Rückfluß gebracht (Innentemperatur ca. 110 - 115°C). Dem Reaktionsgemisch wurde innerhalb von 1 h 0,78 kg (7,96 mol) konz. Schwefelsäure zugetropft und der Ansatz 15 h unter Rühren und Auskreisen von W bei dieser Temperatur gehalten. Nach dieser Zeit lag der durch GC bestimmte Gehalt an unumgesetzten Tocopherol bei 4,5 bis 5 %.
Anschließend wurde das Reaktionsgemisch auf Temperaturen von -5 bis +5°C abgekühlt, die dabei kristallisierende überschüssige Sorbinsäure abfiltriert und das resultierende Filtrat weiter aufgearbeitet.

Dazu wurde es zunächst mit 40 l W und 2,95 l einer 25 gew.-%igen Natronlauge auf pH 7-8 gestellt und anschließend wurden die Phasen getrennt. Nach der basischen Wäsche erfolgten zwei weitere Wäschen mit W und anschließende Phasentrennung.
Die organische Phase wurde in einem Dünnschichtverdampfer eingeengt, wobei ein dunkelbraunes Öl erhalten wurde. Dieses wurde zur weiteren Aufreinigung in einer Molekulardestillation bei ca 0,01 mbar und 210 - 220°C destilliert. Durch die Destillation erhielt man ein transparentes, gelbes Öl mit einem Gehalt an Tocopherylsorbat von > 96 % bei einem Gehalt von freiem Tocopherol von unter 2 %. Die Ausbeute betrug nach der Destillation ca. 80 % der Theorie.

### Beispiel 3

22,4 g (0,05 mol) d,l-α-Tocopherol und 8,8 g (0,08 mol) all-E-Sorbinsäure wurden in einem Gemisch aus 100 ml 1,2-Propylen-carbonat und 100 ml n-Octan gelöst, das erhaltene Reaktionsgemisch mit 4 Tropfen konz. Schwefelsäure versetzt und anschließend für 12 h unter Rückfluß zum Sieden (Innentemperatur 125°C) erhitzt. Während dieser Veresterungsreaktion trennte man dauernd die im Wasserausscheider abgeschiedene Unterphase aus Propylencarbonat und W ab, während das Octan ständig in das Reaktionsgefäß zurückfloß. Das abgetrennte Propylencarbonat wurde dabei ständig durch wasserfreies ersetzt. Nach den 12 h betrug gemäß HPLC (100 % Methanol als Laufmittel, UV-Detektor bei 280 nm, C₃₀-Säule) das Verhältnis von Tocopherol zu Tocopherylsorbat 3/97.

Man kühlte auf Raumtemperatur (RT) ab, trennte die Oberphase ab, wusch diese Octanphase mit verdünnter Sodalösung und W, destillierte das Octan unter vermindertem Druck ab und erhielt so 24,8 g reines all-E-Tocopherylsorbat. Das entspricht einer Ausbeute von 91 % der Theorie.

### Beispiel 4

a) Umsetzung von d,l-α-Tocopherol mit 1 + 1 Mol Sorbinsäure pro Mol Tocopherol in Octan/1,2-Propylen-carbonat
22,4 g (0,05 mol) reines d,1-α-Tocopherol wurden in einem Gemisch aus 100 ml Octan und 100 ml 1,2-Propylen-carbonat auf 100°C erhitzt und dann mit 5,83 g (0,05 mol) reiner Sorbinsäure und 2 Tropfen konz. Schwefelsäure versetzt. Anschließend erhitzte man schnell auf 125°C Innentemperatur und destillierte dann langsam innerhalb von 5 h wasserhaltiges Lösungsmittelgemisch in einen Phasenscheider, wobei die unpolare Octan-Oberphase laufend in das Reaktionsgefäß zurückfloß, während die aus W und Propylencarbonat bestehende polare Unterphase abgetrennt, gesammelt und aufgearbeitet wurde. Die abdestillierte Menge Propylencarbonat wurde im Reaktionsgefäß fortlaufend durch frisches wasserfreies Propylencarbonat ergänzt. Nach 1 h Reaktionszeit ab Reaktionsbeginn und nach einer weiteren h wurden jeweils noch 2,9 g (0,025 mol) reine Sorbinsäure zum Reaktionsgemisch gegeben. Die Umsetzung wurde durch HPLC-Analyse verfolgt:

| Reaktions-Zeit [h] | Tocopherol [F1.%] | Sorbat [F1.%] | Bemerkungen |
|---|---|---|---|
| 1 | 36 | 64 | +2,5 g Sorbinsäure |
| 2 | 17 | 83 | +2,5 g Sorbinsäure |
| 3 | 5 | 95 | |
| 4 | 1,4 | 98,6 | |
| 5 | 1 | 99 | |

Nach insgesamt 5 h kühlte man auf RT ab und trennte die Phasen. Die Unterphase wurde mit 50 ml Octan gewaschen und die vereinigten Octanphasen dann mit 100 ml einer 5 gew.-%igen wässrigen Sodalösung, 100 ml einer 5 gew.-%igen wässrigen Essigsäure und 100 ml W gewaschen. Nach dem Trocknen über MgSO₄ destillierte man das Octan ab und destillierte dann den Rückstand in einem Dünnschichtverdampfer bei 200°C/0,01 mbar Druck. Man erhielt 22,96 g reines d,l- α-Tocopherylsorbat. Dies entspricht einer Ausbeute von 84 % der Theorie.
b) zum Vergleich
Umsetzung von dl-α-Tocopherol mit 1+1 Mol Sorbinsäure pro Mol Tocopherol in Octan
Man arbeitete wie unter a) beschrieben, verwendete jedoch anstelle von dem dort beschriebenen Gemisch aus 100 ml Octan und 100 ml 1,2-Propylen-carbonat nur 200 ml Octan. Auch hierbei wurde der Umsatz des Tocopherols durch HPLC-Analyse verfolgt:

| Reaktions-Zeit [h] | Tocopherol [Fl.%] | Tocopheryl-Sorbat [Fl.%] | Bemerkungen |
|---|---|---|---|
| 1 | 80 | 20 | +2,9 g Sorbinsäure |
| 2 | 73 | 27 | +2,9 g Sorbinsäure |
| 3 | 63 | 37 | |
| 4 | 55 | 45 | |
| 5 | 50 | 51 | |

Die Oberphase floß dauernd ins Reaktionsgefäß zurück, ca. 0,5 ml Unterphase wurden verworfen.

Anschließend wusch man die Reaktionslösung zweimal mit je 200 ml einer 5 gew.-%igen Sodalösung, dann mit einer 5 gew.-%igen wässrigen Essigsäure und W und trocknete. Man destillierte das Octan ab und erhielt 23,1 g eines Rückstands, der zu 12,8 g aus Tocopherol und 10,3 g Tocopherylsorbat bestand. Die Substanzen konnten durch Destillation nicht getrennt werden.

### Beispiele 5, 6 und 7

### Veresterung von Tocopherol mit Sorbinsäure, 1-n-Hexansäure und Ölsäure in Octan/1,2-Propylen-carbonat

Jeweils 89,5 g (0,2 mol) reines d,l-α-Tocopherol (Reinheit 98,5 % wurden in einem Gemisch aus 400 ml 1,2-Propylen-carbonat und 500 ml Octan vorgelegt. Ca. 100 ml Octan wurden abdestilliert und 0,2 mol der aus der folgenden Tabelle ersichtlichen Carbonsäure und die aus der Tabelle ersichtliche Katalysatorsäure in der in der Tabelle angegebenen Menge zugegeben. Man erhitzte auf die in der Tabelle angegebene Innentemperatur und destillierte dann langsam ein Wasser/Octan/Propylencarbonat-Gemisch in einen Phasenscheider ab. Die unpolare Octan-Oberphase lief aus dem Phasenscheider fortlaufend in das Reaktionsgefäß zurück, während die Wasser und Propylencarbonat enthaltende Unterphase zwecks Aufarbeitung gesammelt wurde. Trockenes Propylencarbonat wurde in dem Maße in den Reaktor eingetragen, wie Propylencarbonat abdestillierte. 2 h nach Reaktionsbeginn wurden weitere 0,1 mol der in der Tabelle angegebenen Carbonsäure zugegeben.

Nach der in der Tabelle angegebenen Reaktionszeit wurde das Reaktionsgemisch abgekühlt, die Phasen getrennt und die Unterphase mit 200 ml Octan extrahiert. Die Octanphasen wurden wie oben beschrieben mit 5 Gew.-%iger Sodalösung (in den Beispielen 6 und 7 mit 4 Gew.-%igen Natronlauge), verdünnter Essigsäure und W gewaschen und destilliert. Die erzielten Ausbeuten sind in der folgenden Tabelle angegeben.

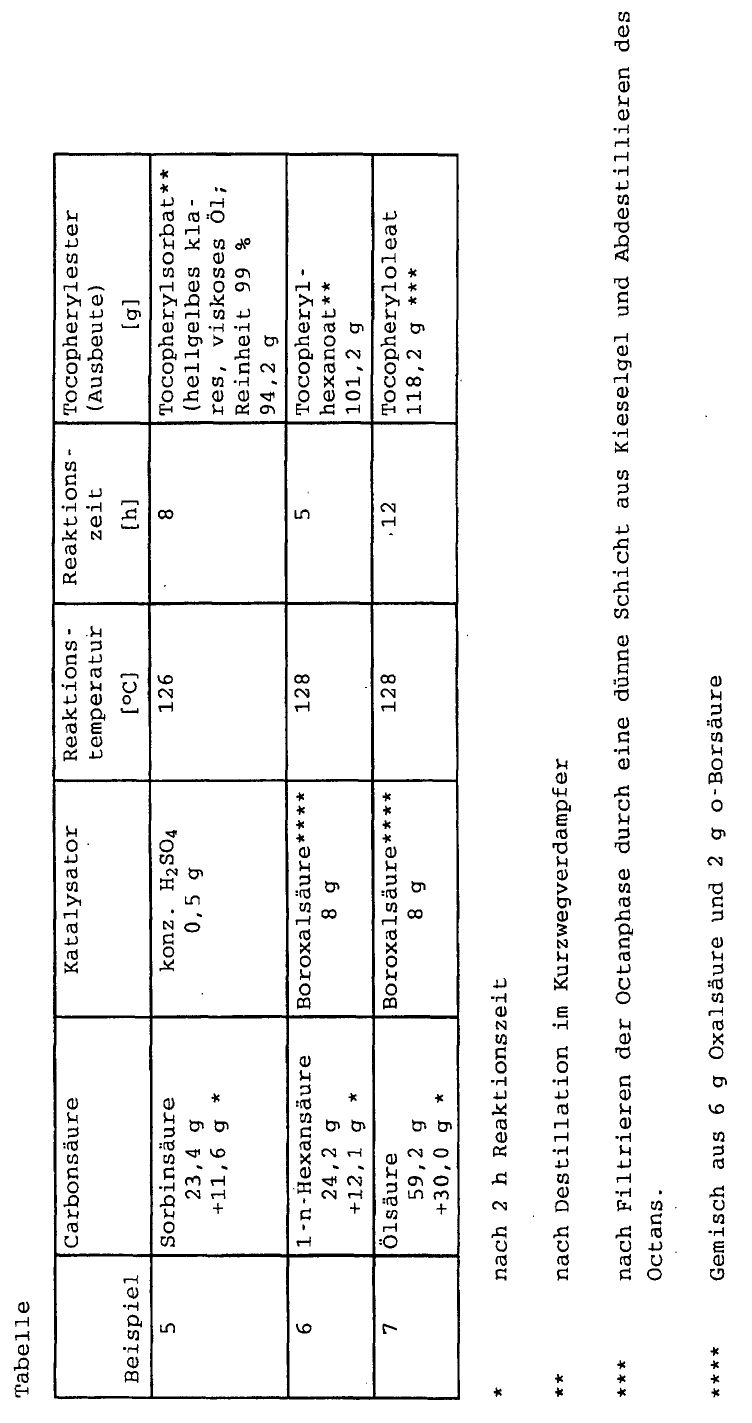

### Beispiel 8

### Veresterung von Tocopherol mit Palmitinsäure

22,4 g d,l-α-Tocopherol wurden zusammen mit 100 ml (120 g) 1,2-Propylen-carbonat und 100 ml Octan auf 125°C erhitzt. Anschließend gab man 13,3 g (0,05 mol) Palmitinsäure und 2 g Boroxalsäure , d. h. eine Mischung aus 0,5 g Borsäure und 1,5 g Oxalsäure, zu und destillierte langsam ein wasserhaltiges Azeotrop ab. Während die unpolare Octan-Oberphase kontinuierlich in das Reaktionsgefäß zurückfloß, wurde die wässrige Propylencarbonat-Unterphase separat zum Aufarbeiten gesammelt. Die abdestillierte Menge an Propylencarbonat wurde im Reaktionsgefäß kontinuierlich ersetzt.
1 h nach Reaktionsbeginn gab man weitere 6,7 g (0,025 mol) Palmitinsäure zu und destillierte weiter.
Nach 7 h hatte sich das Tocopherol zu 97 % in den Palmitinsäureester umgesetzt.
Man kühlte ab, trennte die Phasen und wusch die polare Unterphase mit 50 ml Octan. Die vereinigten Octanphasen wurden mit 5 gew.-%iger Sodalösung, Methanol, 5 gew.-%iger Essigsäure und W gewaschen und getrocknet. Man destillierte das Octan ab und erhielt 29,8 g eines rasch erstarrenden Öls bestehend aus reinem Tocopherylpalmitinsäureester enthaltend nur 0,8 g an unverestertem Tocopherol.

### Beispiel 9

11,1 g reines d,l-α-Tocopherol wurden in einem Gemisch aus 75 ml 1,2-Propylen-carbonat und 75 ml n-Heptan gelöst. Die Lösung wurde nach Erhitzen auf eine Innentemperatur von 102°C mit 6 g Essigsäure und 2 Tropfen (ca. 0,11 g) konz. Schwefelsäure versetzt. Es wurde unter langsamem Abdestillieren eines azeotropen Gemisches aus Wasser, n-Heptan und Propylencarbonat erhitzt, wobei das Heptan aus dem Phasenabscheider fortlaufend in das Reaktionsgefäß zurückgelangt, während die Wasser und Propylencarbonat enthaltende Unterphase zwecks Aufarbeitung gesammelt wurde, und soviel trockenes Propylencarbonat in das Reaktionsgefäß zudosiert wurde, wie Propylencarbonat aus dem Reaktionsgemisch abdestillierte.

Nach 12 h waren 98 % des eingesetzten Tocopherols verestert. Man kühlte auf RT ab, trennte die Heptan-Oberphase ab, wusch sie 2 mal mit verdünnter Sodalösung, destillierte das Heptan ab und fraktionierte den Rückstand im Hochvakuum.
Es wurden 9,1 g reines Tocopherylacetat erhalten.

## Patentansprüche

1. Verfahren zur Herstellung von Tocopherylestern oder Tocotrienylestern durch säurekatalysierte Veresterung von einem Tocopherol oder einem Tocotrienol in einem Lösungsmittel unter Rühren bei erhöhter Temperatur, **dadurch gekennzeichnet, daß** man
a) die Veresterung mit der entsprechenden Carbonsäure durchführt,
b) die Veresterung mit 2,5 bis 6 Mol der Carbonsäure in einem aliphatischen, cycloaliphatischen oder aromatischen Kohlenwasserstoff mit einem Siedebereich von 80 bis 200°C, vorzugsweise 110 bis 130°C, oder aber mit 1,0 bis 2,5 Mol der Carbonsäure in einem zweiphasigen Gemisch aus einem aliphatischen oder cycloaliphatischen Kohlenwasserstoff mit einem Siedebereich von 80 bis 200°C und einem cyclischen Carbonat der allgemeinen Formel II oder einem γ-Lacton der allgemeinen Formel III 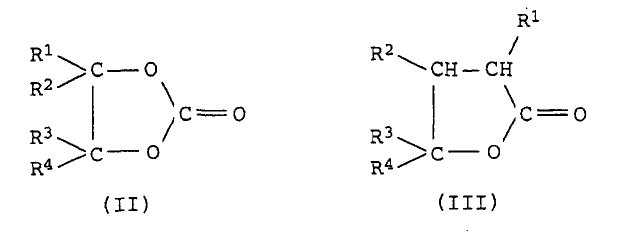 in denen die Reste R¹, R² und R³ für H oder C₁bis C₄-Alkyl stehen und R⁴ für H, C₁- bis C₄-Alkyl, Phenyl oder Methoxymethyl steht, als Lösungsmittel durchführt,
c) eine nicht oxidierende starke anorganische oder organische Säure in katalytischen Mengen als sauren Katalysator verwendet und
d) das bei der Reaktion gebildete Wasser während der Umsetzung fortlaufend azeotrop abdestilliert.

2. Verfahren gemäß Anspruch 1, **dadurch gekennzeichnet, daß** man das Tocopherol mit Essigsäure, Propionsäure, 1-n-Heptansäure, Sorbinsäure, Palmitinsäure, Stearinsäure, Ölsäure oder Linolsäure als Carbonsäure verestert.

3. Verfahren gemäß Anspruch 1, **dadurch gekennzeichnet, daß** man Schwefelsäure, Boroxalsäure oder Benzol- oder Toluolsulfonsäure als sauren Katalysator verwendet.

4. Verfahren gemäß Anspruch 1, **dadurch gekennzeichnet, daß** man zur Herstellung von einem Tocopherylsorbat
a) 2,5 bis 6 Mol, Sorbinsäure pro Mol Tocopherol verwendet und
b) die Umsetzung in einem Kohlenwasserstoff mit einem Siedebereich von 80 bis 200°C bei Temperaturen von 110 bis 130°C durchführt.

5. Verfahren gemäß Anspruch 4, **dadurch gekennzeichnet, daß** man zur Herstellung von einem all-E-Tocopherylsorbat
a) 4,5 bis 5,5 Mol Sorbinsäure pro Mol Tocopherol verwendet und
b) die Umsetzung in Petroleumbenzin mit einem Siedebereich von etwa 80 bis 140°C bei Temperaturen von 113°C bis 120°C durchführt.

6. Verfahren gemäß Anspruch 4 oder 5, **dadurch gekennzeichnet, daß** man das Reaktionsgemisch nach der Veresterungsreaktion auf Temperaturen von 0 bis 5°C abkühlt und die dabei auskristallisierende überschüssige Sorbinsäure abfiltriert, bevor das Reaktionsgemisch in an sich bekannter Weise aufgearbeitet wird.

7. Verfahren gemäß Anspruch 1, **dadurch gekennzeichnet, daß** man zur Herstellung von einem Tocopherylsorbat
a) etwa 1,0 bis 2,5 Mol Sorbinsäure pro Mol Tocopherol verwendet und
b) die Umsetzung in einem Gemisch aus einem aliphatischen oder cycloaliphatischen Kohlenwasserstoff mit einem Siedebereich von 80 bis 200°C und Propylencarbonat oder γ-Butyrolacton durchführt.

8. Verfahren gemäß Anspruch 1, **dadurch gekennzeichnet, daß** man zur Herstellung von einem Tocopherylsorbat
a) etwa 1,5 Mol Sorbinsäure pro Mol Tocopherol verwendet und
b) die Umsetzung in einem Gemisch aus einem Octan und Propylencarbonat oder γ-Butyrolacton bei Temperaturen von etwa 100 bis 150°C durchführt.

9. Verfahren gemäß Anspruch 8, **dadurch gekennzeichnet, daß** man das Tocopherol und die Sorbinsäure in dem Gemisch aus einem Octan und 1,2-Propylen-carbonat oder γ-Butyrolacton vorlegt, konzentrierte Schwefelsäure als Katalysator zutropft, dann das Reaktionsgemisch für 5 bis etwa 15 Stunden zum Sieden unter Rückfluß erhitzt, wobei das bei der Reaktion gebildete Wasser azeotrop abdestilliert und in einem Abscheider fortlaufend in Form einer als Unterphase anfallenden Lösung in Propylencarbonat oder γ-Butyrolacton aus dem aufgefangenen Kondensat abgetrennt und das Propylencarbonat oder γ-Butyrolacton durch wasserfreies Propylencarbonat oder γ-Butyrolacton ersetzt wird, und daß man letztlich das gewünschte Tocopherylsorbat aus der Octanphase in an sich bekannter Weise isoliert.

## Claims

1. A process for preparing tocopheryl esters or tocotrienyl esters by acid-catalyzed esterification of a tocopherol or a tocotrienol in a solvent while stirring at elevated temperature, which comprises
a) carrying out the esterification with the appropriate carboxylic acid,
b) carrying out the esterification with from 2.5 to 6 mol of the carboxylic acid in an aliphatic, cycloaliphatic or aromatic hydrocarbon boiling in the range from 80 to 200°C, preferably 110 to 130°C,
or else with from 1.0 to 2.5 mol of the carboxylic acid in a two-phase mixture consisting of an aliphatic or cycloaliphatic hydrocarbon boiling in the range from 80 to 200°C and a cyclic carbonate of the formula II or a γ-lactone of the formula III 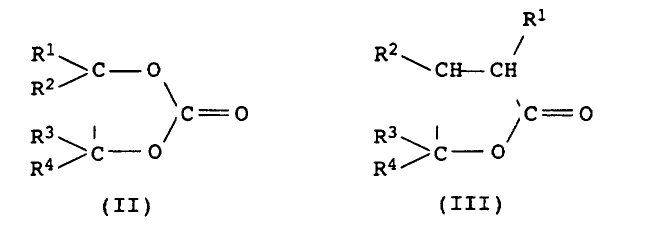 where R¹, R² and R³ are each H or C₁-C₄-alkyl, and R⁴ is H, C₁-C₄-alkyl, phenyl or methoxymethyl, as solvent,
c) using a nonoxidizing strong inorganic or organic acid in catalytic amounts as acid catalyst, and
d) continuously removing the water formed in the reaction by azeotropic distillation during the reaction.

2. A process as claimed in claim 1, wherein the tocopherol is esterified with acetic acid, propionic acid, 1-n-heptanoic acid, sorbic acid, palmitic acid, stearic acid, oleic acid or linoleic acid as carboxylic acid.

3. A process as claimed in claim 1, wherein sulphuric acid, boroxalic acid or benzene- or toluenesulfonic acid is used as acid catalyst.

4. A process as claimed in claim 1, wherein a tocopheryl sorbate is prepared by
a) using from 2.5 to 6 mol of sorbic acid per mole of tocopherol and
b) carrying out the reaction at from 110 to 130°C in a hydrocarbon boiling in the range from 80 to 200°C.

5. A process as claimed in claim 4, wherein an all-E-tocopheryl sorbate is prepared by
a) using from 4.5 to 5.5 mol of sorbic acid per mole of tocopherol and
b) carrying out the reaction at from 113°C to 120°C in petroleum spirit boiling in the range from about 80 to 140°C.

6. A process as claimed in claim 4 or 5, wherein the mixture after the esterification reaction is cooled to from 0 to 5°C, and the excess sorbic acid which crystallizes out is filtered off before the reaction mixture is worked up in a conventional way.

7. A process as claimed in claim 1, wherein a tocopheryl sorbate is prepared by
a) using about 1.0 - 2.5 mol of sorbic acid per mole of tocopherol and
b) carrying out the reaction in a mixture of an aliphatic or cycloaliphatic hydrocarbon boiling in the range from 80 to 200°C and propylene carbonate or γ-butyrolactone.

8. A process as claimed in claim 1, wherein a tocopheryl sorbate is prepared by
a) using about 1.5 mol of sorbic acid per mole of tocopherol and
b) carrying out the reaction in a mixture of an octane and propylene carbonate or γ-butyrolactone at about 100 to 150°C.

9. A process as claimed in claim 8, wherein the tocopherol and the sorbic acid are introduced into the mixture of an octane and 1,2-propylene carbonate or γ-butyrolactone, concentrated sulphuric acid is added dropwise as catalyst, then the reaction mixture is refluxed for from 5 to about 15 hours, with the water formed in the reaction being removed by azeotropic distillation and being continuously separated out in the form of a solution in propylene carbonate or γ-butyrolactone which results as lower phase from the condensate collected in a trap and the propylene carbonate or γ-butyrolactone being replaced by anhydrous propylene carbonate or γ-butyrolactone, and where finally the required tocopheryl sorbate is isolated from the octane phase in a conventional way.

## Revendications

1. Procédé pour la préparation d'esters tocophéryliques ou d'esters tocotriényliques par estérification catalysée par un acide d'un tocophérol ou d'un tocotriénol dans un solvant sous agitation à haute température, **caractérisé par le fait que**
a) on procède à l'estérification par l'acide carboxylique correspondant,
b) on procède à l'estérification par 2,5 à 6 mol de l'acide carboxylique dans un hydrocarbure aliphatique, cycloaliphatique ou aromatique bouillant de 80 à 200°C, de préférence de 110 à 130°C,
ou bien par 1,0 à 2,5 mol de l'acide carboxylique dans un mélange à deux phases consistant en un hydrocarbure aliphatique ou cycloaliphatique bouillant de 80 à 200°C et un carbonate cyclique de formule générale II ou une gamma-lactone de formule générale III
dans lesquelles les symboles R¹, R² et R³ représentent H ou des groupes alkyle en C1-C4 et R⁴ représente H, un groupe alkyle en Cl-C4, phényle ou méthoxyméthyle, qui constitue le solvant,
c) on utilise en tant que catalyseur acide un acide minéral ou organique fort non oxydant en quantité catalytique et
d) on élimine en continu en cours de réaction l'eau formée dans la réaction par distillation azéotropique.

2. Procédé selon la revendication 1, **caractérisé par le fait que** l'acide carboxylique utilisé pour l'estérification du tocophérol est l'acide acétique, l'acide propionique, l'acide 1-n-heptanoïque, l'acide sorbique, l'acide palmitique, l'acide stéarique, l'acide oléique ou l'acide linoléique.

3. Procédé selon la revendication 1, **caractérisé par le fait que** le catalyseur acide utilisé est l'acide sulfurique, l'acide boroxalique ou l'acide benzène- ou toluène-sulfonique.

4. Procédé selon la revendication 1, **caractérisé par le fait que**, pour la préparation d'un sorbate de tocophéryle,
a) on utilise 2,5 à 6 mol d'acide sorbique par mol de tocophérol,
b) on exécute la réaction dans un hydrocarbure bouillant de 80 à 200°C à des températures de 110 à 130°C.

5. Procédé selon la revendication 4, **caractérisé par le fait que**, pour la préparation d'un sorbate de tout-E-tocophéryle
a) on utilise 4,5 à 5,5 mol d'acide sorbique par mol de tocophérol et
b) on exécute la réaction dans l'essence de pétrole bouillant d'environ 80 à 140°C à des températures de 113 à 120°C.

6. Procédé selon la revendication 4 ou 5, **caractérisé par le fait que**, après la réaction d'estérification, on refroidit le mélange de réaction à une température de 0 à 5°C et on filtre l'excès d'acide sorbique qui cristallise avant de traiter ensuite le mélange de réaction de manière connue en soi.

7. Procédé selon la revendication 1, **caractérisé par le fait que**, pour la préparation d'un sorbate de tocophéryle
a) on utilise environ 2,0 à 2,5 mol d'acide sorbique par mol de tocophérol et
b) on exécute la réaction dans un mélange d'un hydrocarbure aliphatique ou cycloaliphatique bouillant de 80 à 200°C et de carbonate de propylène ou de gamma-butyrolactone.

8. Procédé selon la revendication 1, **caractérisé par le fait que**, pour la préparation d'un sorbate de tocophéryle,
a) on utilise environ 1,5 mol d'acide sorbique par mol de tocophérol et
b) on exécute la réaction dans un mélange d'un octane et de carbonate de propylène ou de gamma-butyrolactone à des températures d'environ 100 à 150°C.

9. Procédé selon la revendication 8, **caractérisé par le fait que** l'on introduit le tocophérol et l'acide sorbique dans le mélange d'un octane et du carbonate de 1,2-propylène ou de la gamma-butyrolactone, on ajoute goutte à goutte l'acide sulfurique concentré servant de catalyseur, on chauffe ensuite le mélange de réaction au reflux à l'ébullition pendant 5 à 15 h environ en soumettant l'eau formée dans la réaction à distillation azéotropique et en la séparant du condensat receuilli en continu dans un séparateur sous la forme d'une solution dans le carbonate de propylène ou la gamma-butyrolactone, constituant la phase inférieure, on remplace le carbonate de propylène ou la gamma-butyrolactone par du carbonate de propylène ou de la gamma-butyrolactone anhydre et finalement, on isole le sorbate de tocophéryle recherché de la phase octane de manière connue en soi.
